# EUROPEAN PATENT APPLICATION

(11) **EP 3 608 405 A1**
(43) Date of publication of application: **12.02.2020**
(21) Application number: 17904384.9
(22) Date of filing: 05.04.2017
(51) Int. Cl.: C12N 15/09, C12Q 1/68

(54) **NUCLEIC ACID AMPLIFICATION METHOD AND NUCLEIC ACID ANALYZER**

(71) Applicant: Hitachi High-Technologies Corporation, Minato-ku Tokyo 105-8717 (JP)
(72) Inventor: YOKOI, Takahide, Tokyo 100-8280 (JP); UEMATSU, Chihiro, Tokyo 100-8280 (JP); ITABASHI, Naoshi, Tokyo 100-8280 (JP)
(74) Representative: Strehl Schübel-Hopf & Partner
(86) International application number: PCT/JP2017/014213
(87) International publication number: WO 2018/185871

(57) **Abstract**

Provided are a method and apparatus for forming clusters of amplified nucleic acid fragments without amplification bias, in a regular arrangement on a substrate. In the method according to the present invention, droplets enclosing the template nucleic acid are formed on a substrate that has a plurality of first surfaces having hydrophilicity and a second surface surrounding each of the plurality of first surfaces and being less hydrophilic than the first surfaces. Then, after a nucleic acid amplification reaction is performed in the droplets on the substrate, the droplets are removed and a nucleic acid amplification reaction is further performed on the substrate.

## Description

### Technical Field

The present invention relates to an on-substrate nucleic acid amplification method and an apparatus for nucleic acid analysis.

### Background Art

PTL 1 describes, as a nucleic acid amplification method on a substrate, a method for generating a patterned surface of biomolecules. The method can include the steps of (a) preparing a reagent including (i) an array having non-contiguous features on a surface, the features being separated by interstitial regions of the surface and (ii) a solution having a plurality of different target biomolecules; and (b) reacting the reagent to transport the biomolecules to the features and attach an individual biomolecule to each of the features, wherein an electric field is applied to the interstitial regions to repel the biomolecules from the interstitial regions.

PTL 2 describes a use of an array in which the bottom surface of a receptacle 13 is hydrophilic and the upper surface of a side wall 12 is hydrophobic, as a method of sealing a substance such as nucleic acid on a substrate. It is described that a first solvent 20 containing beads 21 and 21' thus can be more efficiently introduced into the receptacles 13 when the hydrophilic first solvent 20 is used in the step of beads introduction, and further that, because a hydrophobic second solvent 30 used in the step of beads storing can be prevented from entering into the receptacles 13, the hydrophilic first solvent 20 in the receptacles 13 can be coated and hermetically sealed with the hydrophobic second solvent 30 to form droplets (liquid droplets).

### Citation List

### Patent Literature

PTL 1: WO 2013/188582 A
PTL 2: WO 2016/006208 A

### Summary of Invention

### Technical Problem

In a parallel sequencer, in order to improve the ease of analysis, a technique for forming clusters of amplified nucleic acid fragments derived from a single molecule, in a regular arrangement on a substrate, is required.

PTL 1 describes a technique for forming clusters of amplified nucleic acid fragments derived from a single molecule, in a regular arrangement on a substrate. However, because amplification reactions of multiple templates proceed in a non-separated solution, there has been a problem where amplification bias, in which a DNA molecule previously contributing to cluster formation due to differences in the attachment rate of template DNA on the substrate further becomes a template of cluster formation on the peripheral substrate, occurs. Therefore, a technique for amplifying each template in a separated liquid on a substrate is required.

On the other hand, PTL 2 describes a technique for separating substances such as nucleic acid by droplets, in a regular arrangement on a substrate. However, since cluster formation of amplified nucleic acid fragments on the substrate is not realized yet, it has been necessary to arrange clusters of amplified nucleic acid fragments formed by another device on the substrate.

An object of the present invention is to provide a technique for forming clusters of amplified nucleic acid fragments without amplification bias, in a regular arrangement on a substrate.

### Solution to Problem

In order to solve the above problems, according to the present invention, there is provided a nucleic acid amplification method including the steps of:
preparing a substrate that has a plurality of first surfaces having hydrophilicity and a second surface surrounding each of the plurality of first surfaces and being less hydrophilic than the first surfaces, wherein a molecule that specifically binds to a template nucleic acid is fixed or arranged on the first surfaces;
supplying a mixed solution of a sample solution containing the template nucleic acid and a reaction solution containing a nucleic acid amplification substrate and a nucleic acid synthetic enzyme on the substrate to arrange the mixed solution on the first surfaces, and bind the template nucleic acid to the molecule that specifically binds to the template nucleic acid;
supplying a hydrophobic solvent on the substrate to form droplets in which the mixed solution arranged on the first surfaces is enclosed;
performing an amplification reaction of the nucleic acid in the droplets;
removing the hydrophobic solvent from the substrate;
supplying a reaction solution containing a nucleic acid amplification substrate and a nucleic acid synthetic enzyme on the substrate; and
performing an amplification reaction of the nucleic acid.

Furthermore, in order to solve the above problems, according to the present invention, there is provided an apparatus for nucleic acid analysis including a reaction vessel in which a nucleic acid amplification reaction is performed; a sample solution tank for storing a sample solution containing a template nucleic acid; a hydrophobic solvent tank for storing a hydrophobic solvent; and a reaction solution tank for storing a reaction solution containing at least a nucleic acid amplification substrate, wherein the reaction vessel is provided with a first opening and a second opening that are connectable to the sample solution tank, the hydrophobic solvent tank, and the reaction solution tank; a substrate that has a plurality of first surfaces having hydrophilicity and a second surface surrounding each of the plurality of first surfaces and being less hydrophilic than the first surfaces is provided on either a top surface or a bottom surface in the reaction vessel; and a molecule that specifically binds to a template nucleic acid is fixed or arranged on the first surfaces.

### Advantageous Effects of Invention

According to the present invention, it is possible to form clusters of amplified nucleic acid fragments without amplification bias, in a regular arrangement on a substrate. Therefore, it becomes possible to perform efficient and highly accurate nucleic acid amplification in a parallel sequencer and subsequent nucleic acid analysis.

### Brief Description of Drawings

FIG. 1 is a configuration diagram of an apparatus for nucleic acid analysis in Example 1.
FIG. 2A(a) is a diagram showing an example of a substrate having a hydrophilic pattern region in Example 1. FIG. 2A(b) is a diagram showing a cross section of the example of a substrate having a hydrophilic pattern region in Example 1.
FIG. 2B is a diagram showing a specific embodiment of a hydrophilic pattern region in the apparatus for nucleic acid analysis in Example 1.
FIG. 3 shows a schematic diagram of each step of a nucleic acid amplification method of Example 1.
FIG. 4 shows a schematic diagram of each step of a nucleic acid amplification method of Example 2.
FIG. 5 shows a conceptual diagram of a result of the nucleic acid amplification method of Example 1 or 2.
FIG. 6 shows a schematic diagram of each step of a nucleic acid amplification method of Example 3.
FIG. 7 shows a conceptual diagram of a result of the nucleic acid amplification method of Example 3.
FIG. 8 shows a schematic diagram of each step of a nucleic acid amplification method of Example 4.
FIG. 9 is a configuration diagram of an apparatus for nucleic acid analysis in Example 5.
FIG. 10 shows a schematic diagram of each step of a nucleic acid amplification method of Example 5.
FIG. 11 shows a flowchart of the nucleic acid amplification method of Example 5.
FIG. 12 shows a flowchart of the nucleic acid amplification method of Example 5.
FIG. 13 is a configuration diagram of an apparatus for nucleic acid analysis in Example 6 when DNA sequencing is performed.

### Description of Embodiments

Hereinafter, examples will be described with reference to drawings, but the present invention is not limited to these examples.

### Example 1

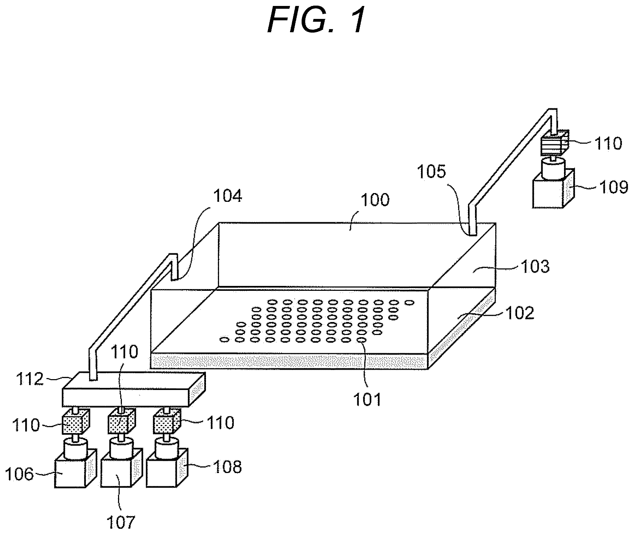

FIG. 1 is a configuration diagram of an example of an apparatus for nucleic acid analysis for performing a nucleic acid amplification method in this example. A flow cell 100 as a reaction vessel, in which a nucleic acid amplification reaction is performed, may be provided with, on its bottom surface, a substrate 102 that has a plurality of first surfaces having hydrophilicity arranged with arbitrary regularity and a second surface that is surrounding each of the plurality of first surfaces and is less hydrophilic than the first surfaces. Hereinafter, the first surfaces are referred to as a hydrophilic pattern region 101. The substrate 102 may also be installed on the upper surface in the flow cell 100. The flow cell 100 may include a first opening 104 and a second opening 105, and supply of any liquid into the flow cell 100 and discharge from the flow cell 100 are possible through the first opening 104 and the second opening 105. An area through which the liquid flows is referred to as a flow cell solution tank 103. A sample solution tank 106, a hydrophobic solvent tank 107, and a reaction solution tank 108 are connected to the first opening 104 through respective valves 110. The drainage tank 109 is connected to the second opening 105 through a valve 110. By opening and closing of each valve 110, control of supply and discharge of liquid into and from the flow cell 100 are possible. Here, a sample solution stored in the sample solution tank 106 may be a solution containing a template nucleic acid. The template nucleic acid may be double-stranded or single-stranded, DNA or RNA, or a hybrid nucleic acid. A reaction solution stored in the reaction solution tank 108 may be a solution containing, for example, a nucleic acid synthetic enzyme, a nucleic acid amplification substrate, and the like. A hydrophobic solvent stored in the hydrophobic solvent tank 107 may be a solvent separated into two layers when mixed with the sample solution and the reaction solution, and preferably has low solubility in the sample solution and the reaction solution and has high resilience. In addition, the specific gravity of the hydrophobic solvent to the sample solution and the reaction solution can be selected, depending on a change in installation form of the substrate 102 on the upper surface or the lower surface of the flow cell 100. For example, aliphatic hydrocarbon (alkane, cycloalkane, squalene, or the like), aromatic hydrocarbon (benzene, toluene, or the like), silicone oil, paraffin oil, fluorocarbon liquid (Fluorinert FC-40 or the like) or the like can be used.

The number of openings to be connected may be increased in accordance with the type(s) of liquid(s) supplied to the flow cell 100, or the number of liquid tanks to be connected to the openings may be increased. Further, by providing a pump 112 in at least one or both of the first opening 104 and the second opening 105, supply and discharge of the solution into and from the flow cell solution tank 103 by pressurization or depressurization are possible. Also, the flow cell solution tank 103 may be configured to be capable of adjusting to a target temperature that is suitable for a nucleic acid amplification reaction and a subsequent enzyme reaction such as nucleotide sequence analysis, and a temperature adjustment mechanism is not shown but may be included in the flow cell 100, or may be included separately from the flow cell 100. Although not shown, each of the sample solution tank 106, the hydrophobic solvent tank 107, the reaction solution tank 108, and the drainage tank 109 may be provided with a temperature control mechanism which is different from that of the flow cell solution tank 103, and can be adjusted to any temperature which is different from the flow cell solution tank 103. In addition, the number of the sample solution tanks 106 and the reaction solution tanks 108 may be increased according to the requirements due to the characteristics of the nucleic acid amplification reaction, such as separation of a reagent for which mixing immediately before use is desirable. Moreover, it is observable (preferably, optically observable) on the substrate 102, and the apparatus for nucleic acid analysis according to the present invention may include an observation unit capable of observing the substrate in the flow cell, although not shown. Furthermore, it is possible to perform a nucleic acid amplification reaction even when the flow cell 100 is vertically inverted.

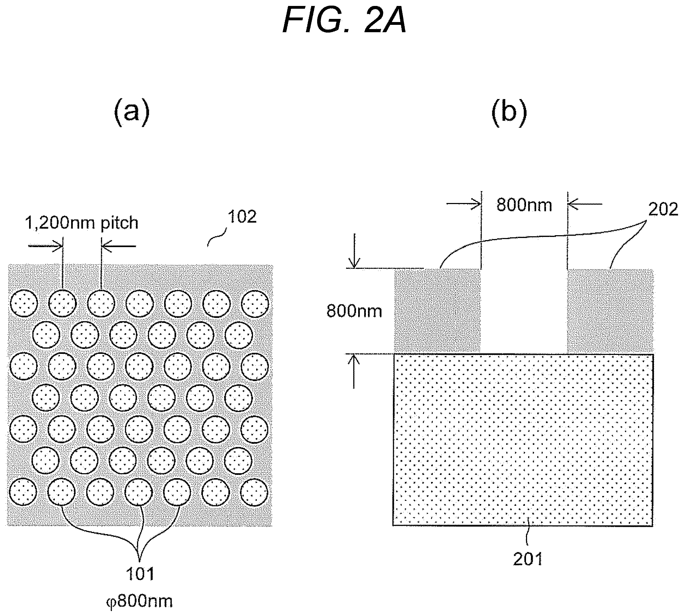

FIG. 2A is a configuration example of a substrate 102 having a hydrophilic pattern region 101. FIG. 2A(a) is an example of a substrate in which a large number of hydrophilic pattern regions 101 with a diameter of 0.8 µm are arranged such that the distance between the centers is 1.2 µm, and the density of hydrophilic pattern regions 101 of this configuration is about 800,000 pieces/mm2. When used in a parallel sequencer, in order to improve nucleotide sequence analysis throughput by arranging clusters of nucleic acid amplification fragments at a high density, the diameter of hydrophilic pattern region 101 may preferably be about 0.5 to 2.0 µm, and the density of hydrophilic pattern region 101 may preferably be about 200,000/mm² or more. It may be preferable to arrange hydrophilic pattern regions regularly, which further improves the throughput of nucleotide sequence analysis and facilitates observation and image processing.

FIG. 2A(b) is an example seen from the cross section of the substrate having the hydrophilic pattern region 101 of (a). A lower layer substrate 201 of the substrate 102 is hydrophilic, and a partition wall 202 having lower hydrophilicity than the lower layer substrate 201 is present on the lower layer substrate 201, thereby forming the hydrophilic pattern region 101 arranged in parallel on the substrate. In the illustrated example, the substrate 102 has a concave structure with a diameter of the hydrophilic pattern region 101 of 0.8 µm and a height of the partition wall 202 of 0.8 µm. The bottommost surface of this concave structure is the hydrophilic pattern region 101 (first surface). Such a configuration may be preferable in terms of ease of formation of droplets and high-density arrangement. The shape of the hydrophilic pattern region 101 is not limited, and may be circular, oval, square or the like. Further, the side surface of the partition wall 202 may be hydrophilic or hydrophobic.

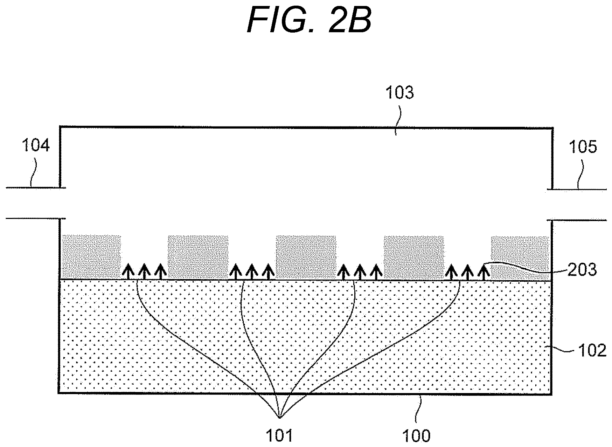

FIG. 2B shows an embodiment of a hydrophilic pattern region in the apparatus for nucleic acid analysis. A molecule 203 for fixing a template nucleic acid is fixed on the substrate (preferably on the first surfaces). In one embodiment, a molecule 203 that specifically binds to the template nucleic acid (that is, an oligonucleotide having complementarity to a part of the template nucleic acid) as a primer for nucleic acid amplification may be fixed or arranged on the hydrophilic pattern region 101 of the substrate. For example, an oligonucleotide can be immobilized on the hydrophilic pattern region 101, or a hydrogel containing an oligonucleotide can be arranged on the hydrophilic pattern region 101. Here, the molecule that specifically binds to the template nucleic acid may vary depending on the type and sequence of the template nucleic acid, and can be routinely designed by those skilled in the art. In another embodiment, a functional group 203 that binds to a primer for nucleic acid amplification may be fixed on the hydrophilic pattern region 101, and the primer for nucleic acid amplification may be added to the sample solution 310 and/or the reaction solution 330 to arrange or fix the nucleic acid amplification primer on the substrate via the functional group after the supply of the sample solution 310 or the reaction solution 330. As such a functional group, for example, avidin-biotin binding, APTES-amino group binding can be utilized.

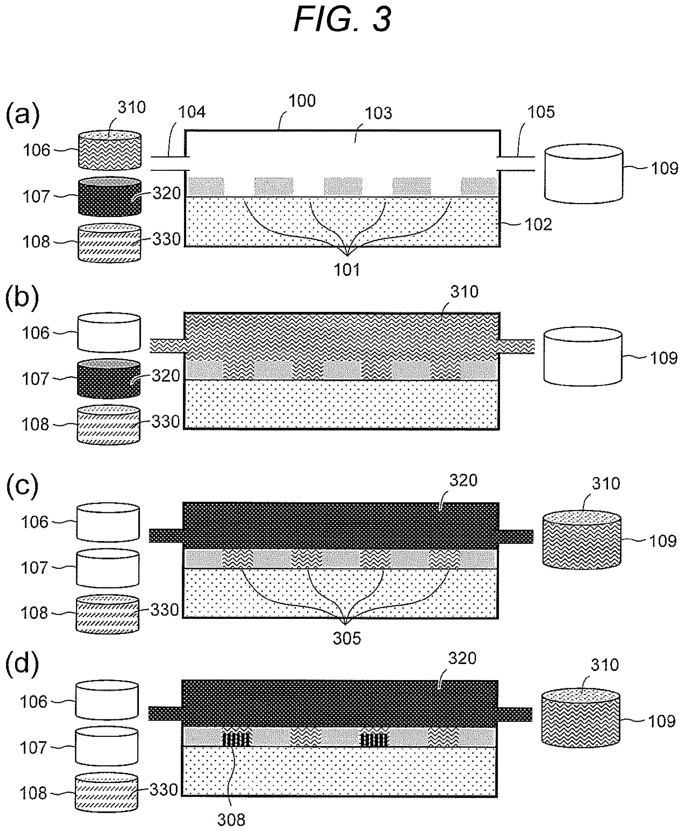

Each step of the on-substrate nucleic acid amplification method in this example will be described using a side sectional diagram of the flow cell 100 shown in FIG. 3. FIG. 3 shows an embodiment in which the sample solution 310 is a reaction solution containing a template nucleic acid (that is, containing a nucleic acid synthetic enzyme, a primer and a substrate).

In the present invention, the template nucleic acid is not particularly limited as long as it is a nucleic acid for which amplification is desired. Examples thereof include messenger RNA (mRNA), non-coding RNA (ncRNA), microRNA, genomic DNA, and fragments thereof, hybrid nucleic acids of DNA and RNA, and the like, and the template nucleic acid may be single-stranded or double-stranded. In a next generation sequencer (NGS), it is preferable to read diverse and uniform sequence clusters when sequencing nucleic acids. Therefore, a template nucleic acid as described above may be introduced by one molecule or less into a plurality of regularly arranged hydrophilic surfaces, and then an amplification reaction may be performed under the condition in which each hydrophilic surface is separated from the others. Thereby, occurrence of bias at the stages of amplification reaction and sequence analysis can be reduced.

FIG. 3(a): The sample solution tank 106 storing the sample solution 310, the hydrophobic solvent tank 107 storing the hydrophobic solvent 320, the reaction solution tank 108 storing the reaction solution 330, and the drainage tank 109 may be prepared. The concentration of the sample solution 310 may be adjusted so that the template nucleic acid in the droplets formed on the hydrophilic pattern region 101 is a single molecule. The nucleic acid synthetic enzyme used for the reaction may be a strand displacement nucleic acid synthetic enzyme used for rolling circle amplification (RCA) or a thermostable nucleic acid synthetic enzyme used for polymerase chain reaction (PCR), and can be freely selected by those skilled in the art according to a means for amplification of the given template nucleic acid. For example, when the template nucleic acid is RNA, a complementary strand synthetic enzyme may be used first. Further, a molecule that specifically binds to the template nucleic acid may be fixed or arranged on the substrate (preferably on the first surfaces) as a primer for nucleic acid amplification.

FIG. 3(b): The sample solution 310 stored in the sample solution tank 106 may be supplied from the sample solution tank 106 to the flow cell solution tank 103 through the first opening 104.

FIG. 3(c): The hydrophobic solvent 320 stored in the hydrophobic solvent tank 107 may be supplied to the flow cell solution tank 103 through the first opening 104, and excess sample solution 310 may be discharged to the drainage tank 109 through the second opening 105, whereby solution replacement in the flow cell solution tank 103 may be performed. As a result of this solution replacement, the sample solution 310 may remain in the plurality of hydrophilic pattern regions 101 arranged on the substrate 102, and droplets 305 separated by the hydrophobic solvent 320 and the partition wall 202 may be formed on the substrate 102. By being separated in the droplets as above, it is possible to prevent contamination due to diffusion of the template nucleic acid enclosed in the droplets and also prevent evaporation of a small amount of sample solution.

FIG. 3(d): The droplets 305 may contain the template nucleic acid contained in the sample solution 310 and the reaction solution. Therefore, in a state where the droplets 305 are separated by the hydrophobic solvent 320 supplied to the flow cell solution tank 103, a nucleic acid amplification reaction in the droplets 305 may proceed by warming the flow cell solution tank 103 by temperature adjustment function. The nucleic acid amplification reaction may preferably be rolling circle amplification (RCA) or polymerase chain reaction (PCR).

In the present invention, the concentration of the template nucleic acid contained in the sample solution 310 may be a concentration at which droplets enclosing one molecule or less of the template nucleic acid are formed on the substrate, i.e., a concentration at which one molecule per droplet is expected. Therefore, it may be preferable to dilute the sample solution 310 containing the template nucleic acid to that concentration. Thus, after the present nucleic acid amplification step, a hydrophilic pattern region having amplified nucleic acid fragments 308 and a hydrophilic pattern region having no amplified nucleic acid fragment 308 may coexist. By utilizing an oligonucleotide previously immobilized on the hydrophilic pattern region 101 or a hydrogel containing the arranged oligonucleotide as a primer for nucleic acid amplification, the amplified nucleic acid fragments 308 may be immobilized on the hydrophilic pattern region 101 simultaneously parallel with the nucleic acid amplification in the droplets 305. In addition, a fixing method of the nucleic acid amplification fragments to the substrate is not limited to the above, and various methods utilized in this technical field may be applied. Further, at least a part of the amplified nucleic acid fragments 308 in the droplets may be fixed to the hydrophilic pattern region 101.

This makes it possible to amplify each template in the separated droplets 305 on the substrate 102, so that it is possible to realize formation of clusters of the amplified nucleic acid fragments 308 without amplification bias, in a regular arrangement on the substrate.

### Example 2

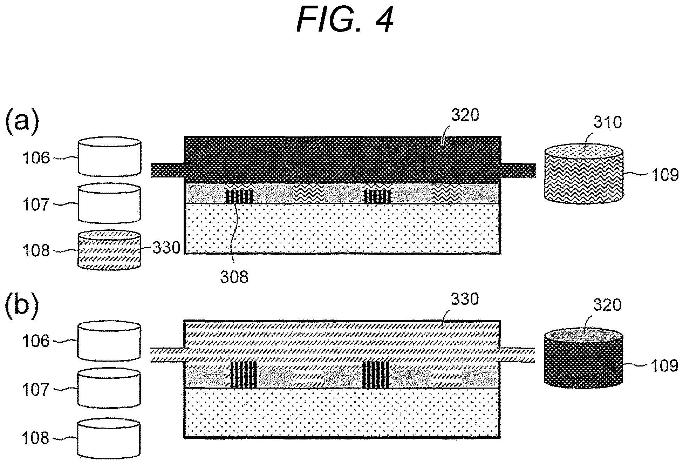

In this example, as a technique for improving the amount of nucleic acid amplification of clusters of amplified nucleic acid fragments on a substrate, steps illustrated in FIG. 4 are performed following the steps illustrated in FIG. 3. That is, after the amplification reaction of the nucleic acid is performed in the droplets, the reaction solution containing at least the amplification reaction substrate may be supplied onto the substrate to perform a further amplification reaction of the nucleic acid.

FIG. 4(a): It is a side sectional diagram of the flow cell 100 in which the nucleic acid amplification reaction is performed in the droplets formed on the substrate 102, and is the same as that shown in FIG. 3(d).

FIG. 4(b): The reaction solution 330 stored in the reaction solution tank 108 may be supplied into the flow cell solution tank 103 through the first opening 104, and the hydrophobic solvent 320 may be discharged to the drainage tank 109 through the second opening 105, whereby solution replacement in the flow cell solution tank 103 may be performed. The hydrophobic solvent 320 may be removed by suction or the like prior to the supply of the reaction solution 330. Further, the hydrophilic pattern region 101 may be dried by removing the hydrophobic solvent 320 and then drying the surface of the substrate 102, and then the reaction solution 330 may be supplied, whereby efficient supply can be realized. As a result, separation of the amplified nucleic acid fragments 308 by the droplets 305 formed on the hydrophilic pattern region 101 may respectively be eliminated, and the reaction solution 330 may be supplied to the amplified nucleic acid fragment 308. By warming the flow cell solution tank 103 with the temperature adjustment function, the nucleic acid amplification reaction may proceed in the hydrophilic pattern region 101 having the amplified nucleic acid fragments 308. Thus, by performing the nucleic acid amplification reaction in a state in which the separation by the droplets 305 is eliminated, a larger copy number of clusters of the amplified nucleic acid fragments 308 derived from a single molecule which cannot be achieved only by the nucleic acid amplification reaction in the droplets 305 on the substrate 102 may be obtained. In addition, after repeating the step of forming droplets and the step of performing an amplification reaction of the nucleic acid in the droplets one or more times, the reaction solution 330 can be supplied onto the substrate 102 to perform a further amplification reaction of the nucleic acid.

The copy number of amplified nucleic acid fragments when assuming the nucleic acid amplification reaction in FIG. 4(a) and FIG. 4(b) on the substrate 102 shown in FIG. 2A will be described. Here, the nucleic acid is DNA (deoxyribonucleic acid). In this example, the size of recess (hydrophilic pattern region) was set to 0.8 µm in both diameter and depth, and the volume was set to about 0.4 fL. When the concentration of the nucleic acid amplification substrates (four types of deoxynucleotide triphosphates (dATP, dCTP, dGTP, and dTTP)) to be used in the reaction is set to 200 µM each, four types of nucleic acid amplification substrates present in the formed droplets may be about 50,000 molecules each. When assuming that a DNA molecule of 400 bases equally containing 100 bases each of the four types of nucleic acid amplification substrates, 100 molecules for each of the four types of nucleic acid amplification substrate molecules may be consumed per amplification of one molecule of DNA. In the DNA amplification reaction of FIG. 4(a), assuming that all nucleic acid amplification substrate molecules are completely consumed for amplification of target DNA, DNA amplification of 500 molecules from 50,000 molecules/100 molecules may be the upper limit. However, since reduction of the nucleic acid amplification substrate concentration in the DNA amplification reaction and pyrophosphoric acid produced as a by-product of the amplification reaction may cause a reduction in the reaction efficiency, it may substantially be difficult to completely consume the given nucleic acid amplification substrate amount for DNA amplification. Subsequently, when the DNA amplification reaction of FIG. 4(b) is performed, the separation by the droplets is eliminated, and the nucleic acid amplification substrate which has been restricted in nucleic acid amplification in the droplets may be newly supplied on the hydrophilic pattern region 101 on which the amplified nucleic acid fragments derived from a single molecule have been immobilized, a further nucleic acid amplification reaction becomes possible, and a copy number of amplified nucleic acid fragments of about 1000 to 10,000 molecules may be obtained.

### Example 3

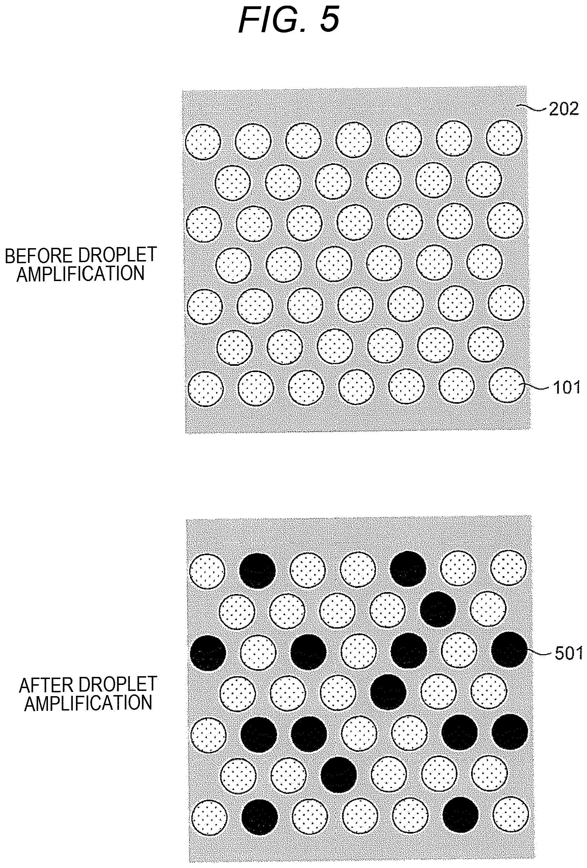

In this example, the hydrophilic pattern region 101 having no amplified nucleic acid fragment 308 in Example 1 or 2 may be reduced, and the rate of a hydrophilic pattern region 501 having the nucleic acid fragment 308 may be improved. As shown in FIG. 5, the rate of a hydrophilic pattern region 501 having nucleic acid fragments amplified from the template nucleic acid to the hydrophilic pattern region 101 before the amplification reaction may be about 30% according to a probability distribution (referred to as Poisson distribution) because it is diluted so that the template nucleic acid to be introduced is one molecule/one droplet.

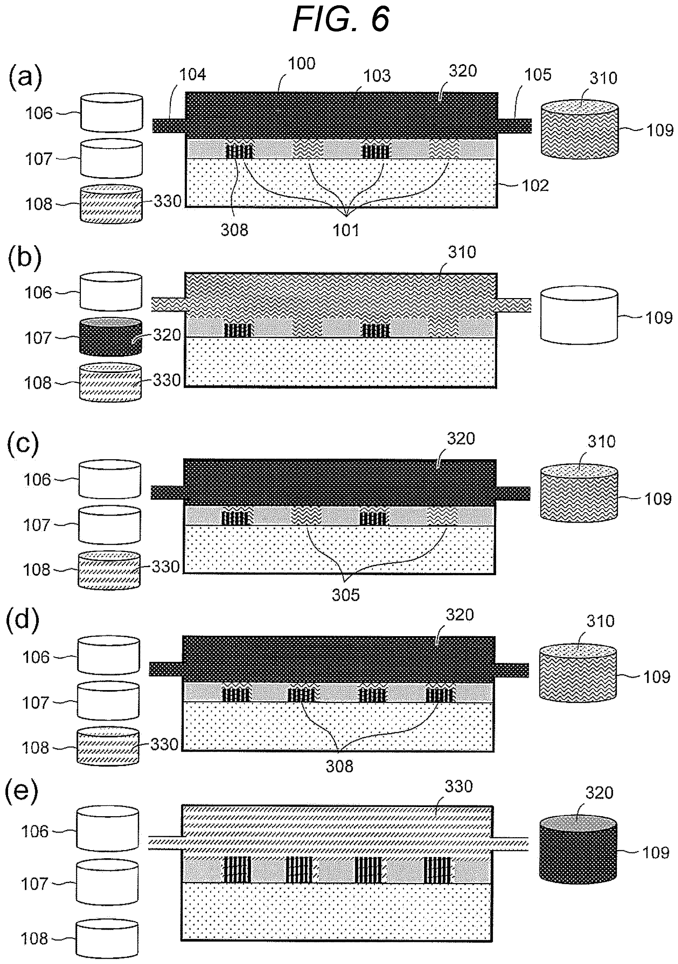

Each step of the on-substrate nucleic acid amplification method in this example will be described with reference to FIG. 6.

FIG. 6(a): The state of FIG. 4(a) is shown. The rate of droplets 305 on the hydrophilic pattern region that possesses the template nucleic acid derived from a single molecule may be about 30% because it follows a probability distribution (referred to as Poisson distribution). Therefore, after performing the nucleic acid amplification reaction shown in FIG. 4(a), the formation of the droplets 305 and the nucleic acid amplification reaction in the droplets 305 shown in FIGS. 6(b) to 6(d) may be repeatedly performed in the flow cell 100.

FIG. 6(b): The sample solution 310 discharged to the drainage tank 109 may be supplied to the flow cell solution tank 103 through the second opening 105, and the hydrophobic solvent 320 in the flow cell solution tank 103 may be discharged to the hydrophobic solvent tank 107 through the first opening 104, whereby solution replacement in the flow cell solution tank 103 may be performed. The hydrophobic solvent 320 may be removed by suction or the like prior to the supply of the sample solution 310. Further, the substrate 102 may be dried after the suction of the hydrophobic solvent 320, and then the reaction solution 330 may be supplied, whereby efficient supply can be realized.

FIG. 6(c): The hydrophobic solvent 320 discharged to the hydrophobic solvent tank 107 may be supplied to the flow cell solution tank 103 through the first opening 104, and excess sample solution 310 that has not used for forming the droplet 305 may be discharged to the drainage tank 109 through the second opening 105, whereby solution replacement in the flow cell solution tank 103 may be performed. As a result, droplets 305 enclosing a template nucleic acid derived from a single molecule may be newly formed on the hydrophilic pattern region 101 having no amplified nucleic acid fragment 308.

FIG. 6(d): In a state where the droplets 305 are separated by the hydrophobic solvent 320 supplied to the flow cell solution tank 103, a nucleic acid amplification reaction in the droplets 305 may proceed by warming the flow cell solution tank 103 by temperature adjustment function. Thereby, clusters of the amplified nucleic acid fragments 308 may be newly formed on the substrate 102.

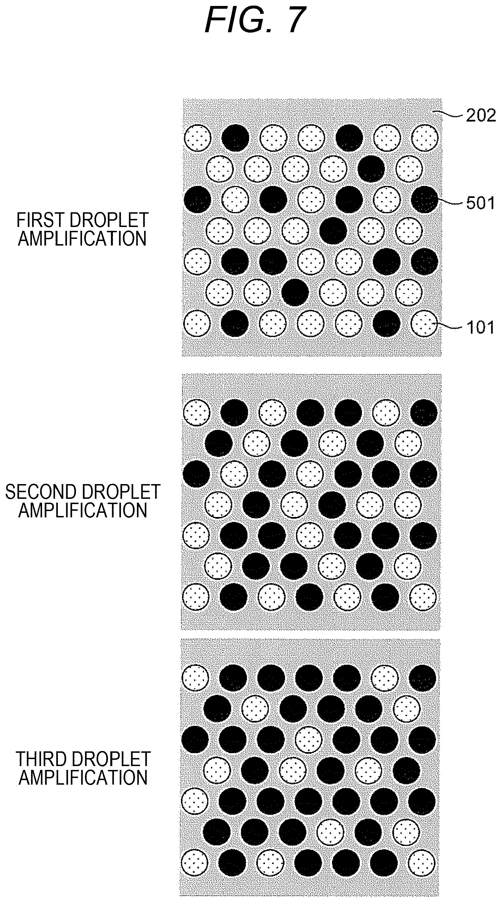

FIG. 6(e): After repeating the steps in FIGS. 6(b) to 6(d) one or more times, the same operation as in FIG. 4(b) may be performed. Thereby, on the substrate 102, the rate of the hydrophilic pattern region 501 in which the clusters of the amplified nucleic acid fragments 308 derived from a single molecule are formed can be improved. Specifically, as shown in FIG. 7, it is expected that about 30% of empty hydrophilic pattern regions 101 will newly become hydrophilic pattern regions 501 having amplified nucleic acid fragments by one repetition of the amplification processing. It may be difficult for the template nucleic acid in the sample solution supplied at the time of repetition of this amplification processing to enter the hydrophilic pattern region already having nucleic acid fragments due to charge repulsion. In addition, even when a new template nucleic acid enters the region already having nucleic acid fragments, the template molecule present in the region and the new template molecule may greatly differ in abundance ratio. Thus, even when the amplification reaction proceeds in the state where entry is allowed, it does not become an obstacle for sequence analysis to be performed later.

In this example, since the sample solution 310 is repeatedly used, the sample solution tank 106 and the drainage tank 109, which store the sample solution 310, may be controlled to any temperature by a temperature adjustment mechanism, which is different from that of the flow cell solution tank 103. Also, in FIGS. 6(a) to 6(d), the sample solution 310 and the hydrophobic solvent 320 may not be reused and a new solution may be prepared, in which case the number of solution tanks to be stored may be added.

### Example 4

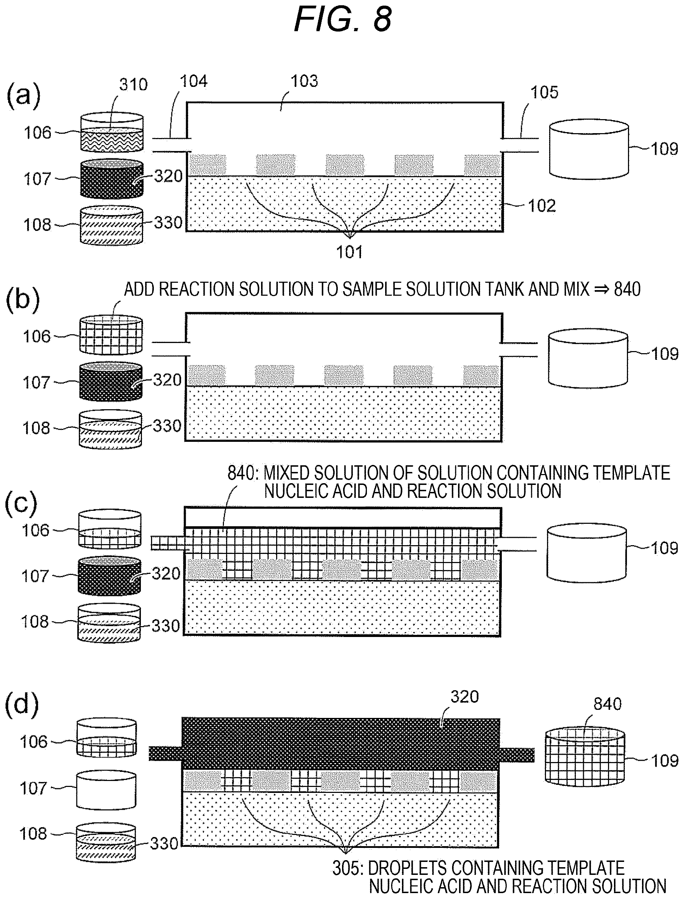

Each step of the on-substrate nucleic acid amplification method in this example will be described using a side sectional diagram of the flow cell 100 shown in FIG. 8. FIG. 8 shows an embodiment in which the sample solution 310 is a solution containing a template nucleic acid, and the sample solution 310 and the reaction solution 330 that is a solution containing a nucleic acid synthetic enzyme, a nucleic acid amplification substrate and the like are mixed in the sample solution tank 106 before formation of droplets by introduction of the hydrophobic solvent 320. Separating the sample solution 310 and the reaction solution 330 before addition to the flow cell 100 may be effective as a means for preventing an undesired DNA amplification reaction in the sample solution.

FIG. 8(a): The sample solution tank 106 storing the sample solution 310, the hydrophobic solvent tank 107 storing the hydrophobic solvent 320, the reaction solution tank 108 storing the reaction solution 330, and the drainage tank 109 may be prepared. The concentration of the sample solution 310 may be adjusted so that the template nucleic acid in the droplets that are a mixture of the sample solution and the reaction solution formed on the hydrophilic pattern region 101 is a single molecule. The nucleic acid synthetic enzyme used for the reaction may be a strand displacement nucleic acid synthetic enzyme used for rolling circle amplification (RCA) or a thermostable nucleic acid synthetic enzyme used for polymerase chain reaction (PCR), and can be freely selected by those skilled in the art according to a means for amplification of the given template nucleic acid. For example, when the template nucleic acid is RNA, a complementary strand synthetic enzyme may be used first. Further, a molecule that specifically binds to the template nucleic acid may be fixed or arranged on the substrate (preferably on the first surfaces) as a primer for nucleic acid amplification.

FIG. 8(b): The reaction solution 330 may be supplied from the reaction solution tank 108 to the sample solution tank 106 to prepare a mixed solution 840 of the sample solution 310 and the reaction solution 330. Although it is also possible to mix the sample solution and the reaction solution in the flow cell solution tank, in order to supply a homogenous mixed solution, it may be desirable to mix them prior to the supply to the flow cell solution tank. It may be preferable not to use the whole reaction solution 330 at this point, since the reaction solution 330 may also be used in the later steps.

FIG. 8(c): The mixed solution 840 may be supplied from the sample solution tank 106 to the flow cell solution tank 103 through the first opening 104.

FIG. 8(d): The hydrophobic solvent 320 stored in the hydrophobic solvent tank 107 may be supplied to the flow cell solution tank 103 through the first opening 104, and excess mixed solution 840 may be discharged to the drainage tank 109 through the second opening 105, whereby solution replacement in the flow cell solution tank 103 may be performed. As a result of this solution replacement, the mixed solution 840 may remain in the plurality of hydrophilic pattern regions 101 arranged on the substrate 102, and droplets 305 separated by the hydrophobic solvent 320 and the partition wall 202 may be formed on the substrate 102. By being separated in the droplets as above, it is possible to prevent contamination due to diffusion of the template nucleic acid enclosed in the droplets and also prevent evaporation of a small amount of sample solution.

Thereafter, as shown in FIG. 3(d), in a state where the droplets 305 are separated by the hydrophobic solvent 320 supplied to the flow cell solution tank 103, a nucleic acid amplification reaction in the droplets 305 may proceed by warming the flow cell solution tank 103 by temperature adjustment function. Also, as shown in Example 2 and FIG. 4, the nucleic acid amplification reaction may be repeatedly performed. At that time, the reaction solution 330 can be supplied from the reaction solution tank 108. Furthermore, as shown in Example 3 and FIG. 6, introduction of the template nucleic acid into the flow cell may be repeatedly performed. At that time, the mixed solution 840 in the sample solution tank 106 or the mixed solution 840 collected in the waste liquid tank 109 can be supplied to the flow cell solution tank 103.

This may make it possible to amplify each template in the separated droplets 305 on the substrate 102, so that it may be possible to realize formation of clusters of the amplified nucleic acid fragments 308 without amplification bias, in a regular arrangement on the substrate. Further, by performing a further nucleic acid amplification reaction after removing the hydrophobic solvent, a copy number of amplified nucleic acid fragments of about 1000 to 10,000 molecules may be obtained.

### Example 5

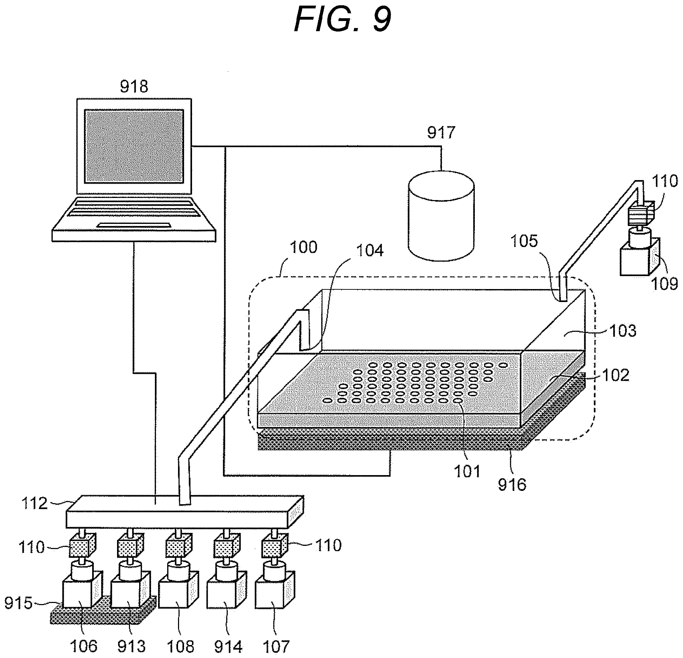

FIG. 9 is a configuration diagram of an example of an apparatus for nucleic acid analysis for performing a nucleic acid amplification method in this example. A flow cell 100, a hydrophilic pattern region 101, a substrate 102, a flow cell solution tank 103, a first opening 104, a second opening 105, a sample solution tank 106, a hydrophobic solvent tank 107, a reaction solution tank 108, a drainage tank 109, a valve 110 and a pump 112 are similar to the configuration of FIG. 1. In the configuration shown in FIG. 9, a solution mixing tank 913 for mixing a sample solution and a reaction solution, a washing solution tank 914 for introducing a washing solution, a temperature control device 915 for controlling the temperature of the sample solution tank and/or the solution mixing tank, a temperature control device 916 for controlling the temperature of the flow cell solution tank 103, an observation unit 917 for observing on the substrate, and a control unit 918 may be provided. The solution mixing tank 913 and the washing solution tank 914 may be connected to the first opening 104 through the respective valves 110. By opening and closing of each valve 110, control of supply and discharge of liquid into and from the flow cell 100 may be possible. As the observation unit, any device can be used as long as it can observe the substrate. For example, an optical microscope, a phase contrast microscope, a fluorescence microscope or the like can be used. With the observation device, a region where the amplified nucleic acid fragment is present, a region where the amplified nucleic acid fragment is not present (i.e., a region where a template nucleic acid was not introduced) and the like may be observed, whereby it may be possible to determine completion of the operation or a repetition of the operation, or determine propriety of transition to subsequent operations. As the control unit, for example, a PC can be used. Similar to the configuration of FIG. 1, it may be possible to perform the nucleic acid amplification reaction even when the flow cell 100 is vertically inverted.

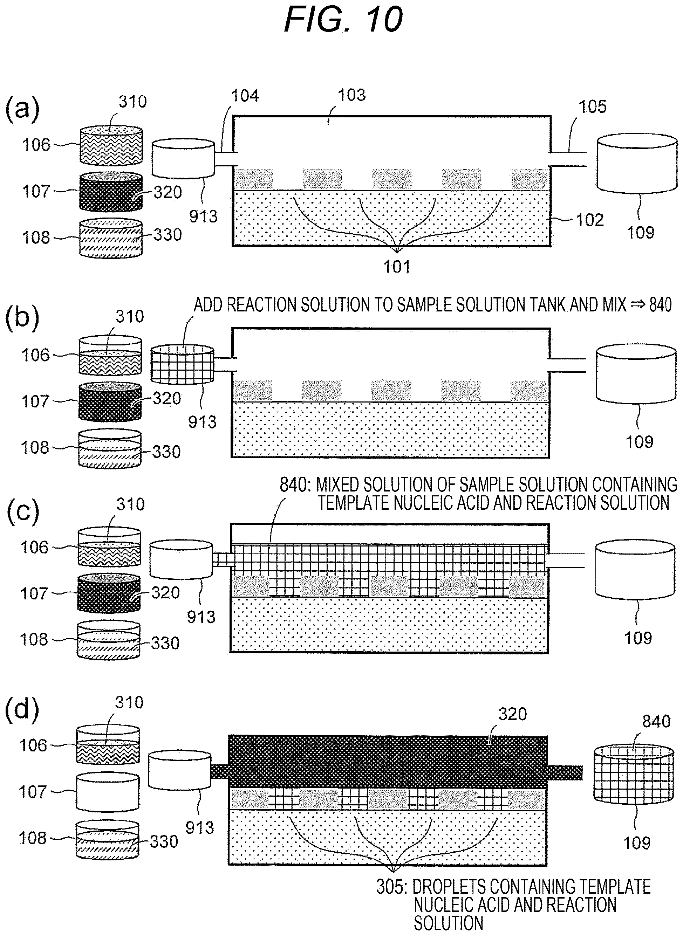

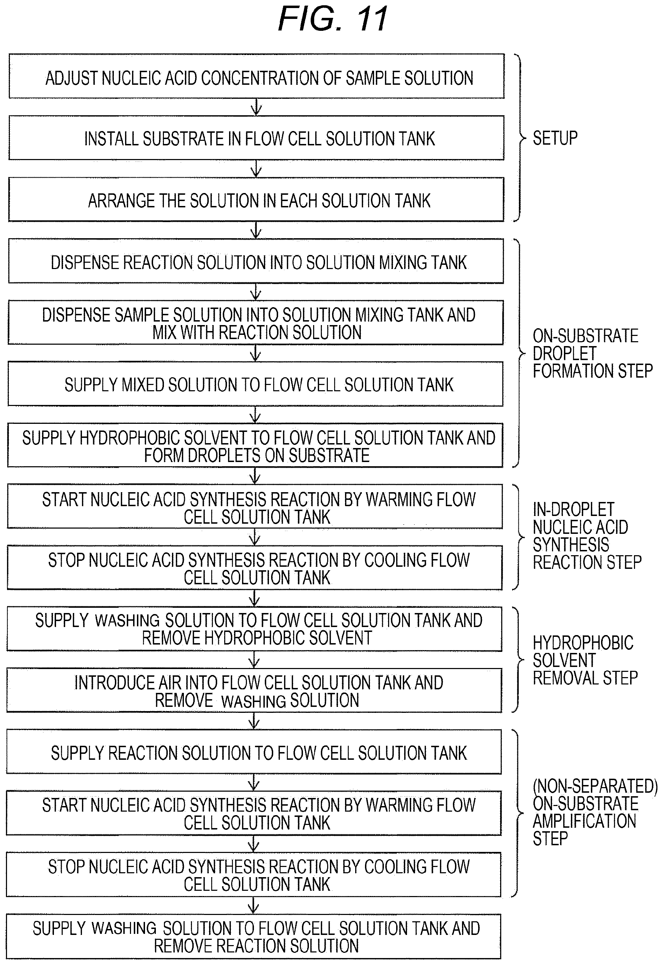

Each step of the on-substrate nucleic acid amplification method in this example will be described using a side sectional diagram of the flow cell 100 shown in FIG. 10. FIG. 10 shows an embodiment in which the sample solution 310 is a solution containing a template nucleic acid, and the sample solution 310 and the reaction solution 330 are mixed in the solution mixing tank 913 before formation of droplets by introduction of the hydrophobic solvent 320. Also, a flowchart of the procedure is shown in FIG. 11.

FIG. 10 (a): The sample solution tank 106 storing the sample solution 310, the hydrophobic solvent tank 107 storing the hydrophobic solvent 320, the reaction solution tank 108 storing the reaction solution 330, the drainage tank 109, and the solution mixing tank 913 may be prepared. The concentration of the sample solution 310 may be adjusted so that the template nucleic acid in the droplets that are a mixture of the sample solution and the reaction solution formed on the hydrophilic pattern region 101 is a single molecule. Specifically, the concentration of the sample solution containing the template nucleic acid may be diluted to one molecule or less/one droplet at the time of mixing with the reaction solution. The nucleic acid synthetic enzyme used for the reaction may be a strand displacement nucleic acid synthetic enzyme used for rolling circle amplification (RCA) or a thermostable nucleic acid synthetic enzyme used for polymerase chain reaction (PCR), and can be freely selected by those skilled in the art according to a means for amplification of the given template nucleic acid. For example, when the template nucleic acid is RNA, a complementary strand synthetic enzyme may be used first. Further, a molecule that specifically binds to the template nucleic acid may be fixed or arranged on the substrate (preferably on the first surfaces) as a primer for nucleic acid amplification. The temperature control device 915 may control the temperature of the sample solution tank 106 and the solution mixing tank 913 from ice temperature to about 4°C.

FIG. 10(b): The sample solution 310 from the sample solution tank 106 and the reaction solution 330 from the reaction solution tank 108 may be dispensed into the solution mixing tank 913 and mixed to prepare a mixed solution 840. The amount of the solution to be mixed may be any amount corresponding to solution composition that is suitable for nucleic acid synthesis, and can be appropriately set by those skilled in the art. It may be preferable not to use all of the sample solution 310 and the reaction solution 330 at this point since they are also used in the later steps.

FIG. 10(c): The mixed solution 840 may be supplied from the solution mixing tank 913 to the flow cell solution tank 103 through the first opening 104. The amount of solution to be supplied may be the amount of liquid sufficient to cover a substrate having recesses (hydrophilic pattern region) installed at the bottom of the flow cell solution tank 103, and it is not necessary to fill the entire flow cell solution tank 103.

FIG. 10(d): The hydrophobic solvent 320 stored in the hydrophobic solvent tank 107 may be supplied to the flow cell solution tank 103 through the first opening 104, and excess mixed solution 840 may be discharged to the drainage tank 109 through the second opening 105, whereby solution replacement in the flow cell solution tank 103 may be performed. As a result of this solution replacement, the mixed solution 840 may remain in the plurality of hydrophilic pattern regions 101 arranged on the substrate 102, and droplets 305 separated by the hydrophobic solvent 320 and the partition wall 202 may be formed on the substrate 102. By being separated in the droplets as above, it may be possible to prevent contamination due to diffusion of the template nucleic acid enclosed in the droplets and also prevent evaporation of a small amount of sample solution (on-substrate droplet formation step). The mixed solution 840 and the hydrophobic solvent 320 may flow into the drainage tank 109, but the mixed solution and the hydrophobic solvent may not be mixed in the drainage tank because one of them is a hydrophobic solvent. Therefore, it may be possible to reuse the mixed solution from the drainage tank. Although it is necessary to supply a hydrophobic solvent in a sufficient amount to discharge the excess mixed solution, it is not necessary to fill the flow cell solution tank 103. When the mixed solution in the drainage tank is reused, the temperature of the drainage tank may be controlled from ice temperature to 4°C.

Thereafter, as shown in FIG. 3(d), in a state where the droplets 305 are separated by the hydrophobic solvent 320 supplied to the flow cell solution tank 103, a nucleic acid synthesis reaction in the droplets 305 may proceed by warming the flow cell solution tank 103 by the temperature control device 916 (in-droplet nucleic acid synthesis reaction step). Although the warming method is not limited, it may be preferable not to disturb observation of amplified nucleic acid clusters on the upper surface of the substrate in the flow cell 100. After nucleic acid amplification, the flow cell solution tank 103 may be cooled to stop the nucleic acid synthesis reaction.

Subsequently, the hydrophobic solvent 320 may be removed from the flow cell solution tank 103 (hydrophobic solvent removal step). The removal of the hydrophobic solvent may be performed by depressurizing or pressurizing the flow cell solution tank 103, may be performed by injecting gas into the flow cell solution tank 103, or may be performed by supplying the washing solution from the washing solution tank 914. The composition of the gas may be to substitutively remove the solution in the flow cell solution tank, and the composition is not particularly limited. The composition of the washing solution may not be particularly limited, but a solution having the same buffer composition as the reaction solution, which does not contain all or part of a nucleic acid synthetic enzyme, a substrate and a primer, may be preferable. When the washing solution is supplied, the washing solution may be removed by introducing gas into the flow cell solution tank 103 or by pressure or reduced pressure.

Also, as shown in Example 2 and FIG. 4, the nucleic acid amplification reaction may be repeatedly performed. At that time, the reaction solution 330 can be supplied from the reaction solution tank 108 to the flow cell solution tank 103. A nucleic acid synthesis reaction may proceed by warming the flow cell solution tank 103 by the temperature control device 916 (non-separated on-substrate amplification step). Since the reaction solution of this step does not contain a template nucleic acid molecule, no new nucleic acid molecule supply to the hydrophilic pattern region 101 onto the substrate may occur. The amount of reaction solution to be supplied is defined as the amount of liquid sufficient to completely cover the bottom surface of the substrate having recesses (hydrophilic pattern region), and when the reaction solution is supplied, the bottom surface of the substrate having a large number of recesses will be one continuous reaction field. Since the substrate amount is limited within the droplets separated by the hydrophobic solvent as described above, the number of amplified copies in the recess may be restricted. However, the restriction of the substrate amount in the recess may be resolved by forming a reaction field in which the droplets are released, allowing synthesis of large numbers of nucleic acid molecules. After nucleic acid amplification, the flow cell solution tank 103 may be cooled to stop the nucleic acid synthesis reaction. If necessary, the washing solution may be supplied from the washing solution tank 914 to the flow cell solution tank 103 to remove the reaction solution.

It may be possible to confirm whether the operation is completed or not while observing the substrate from the observation unit 917, and to determine the repetition of the operation and the transition to the next step. The supply of the solution, warming and cooling of the temperature control device, and observation by the observation unit can be appropriately controlled by the control unit 918.

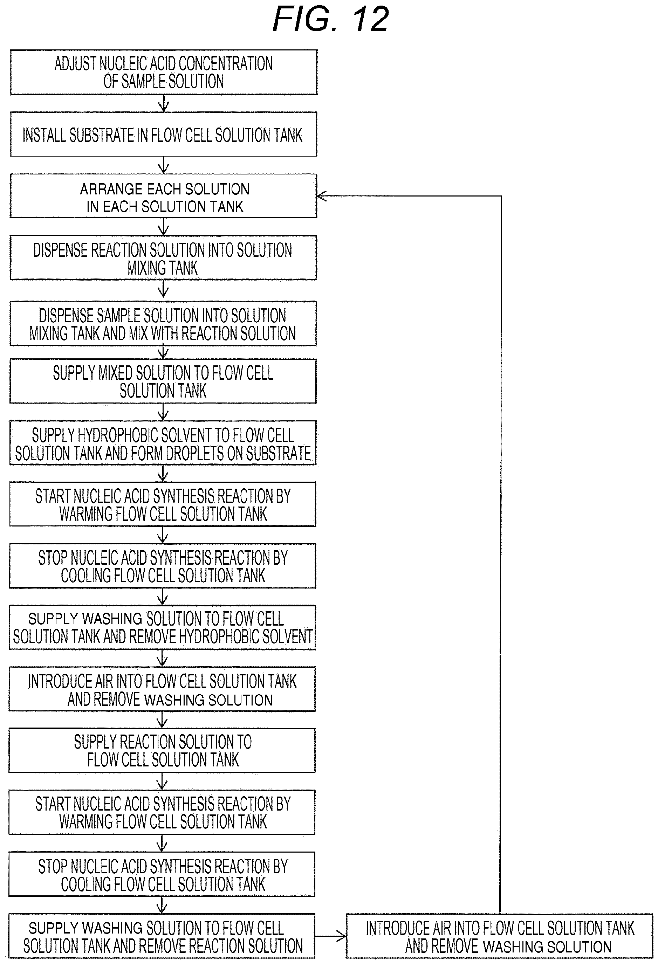

Furthermore, as shown in Example 3 and FIG. 6, introduction of the template nucleic acid into the flow cell may be repeatedly performed. A flowchart of the procedure is shown in FIG. 12. After the non-separated on-substrate amplification step, the washing solution may be supplied from the washing solution tank 914 to the flow cell solution tank 103, the reaction solution may be removed, and subsequently, the washing solution may be removed from the flow cell solution tank 103, followed by repeatedly performing the on-substrate droplet formation step, the in-droplet nucleic acid synthesis reaction step, the hydrophobic solvent removal step and the non-separated on-substrate amplification step. At that time, a mixed solution 840 obtained by mixing the sample solution 310 from the sample solution tank 106 and the reaction solution 330 from the reaction solution tank 108 in the solution mixing tank 913, or a mixed solution 840 collected in the waste liquid tank 109 can be supplied to the flow cell solution tank 103.

This may make it possible to amplify each template in the separated droplets 305 on the substrate 102, so that it is possible to realize formation of clusters of the amplified nucleic acid fragments 308 without amplification bias, in a regular arrangement on the substrate. Further, by performing a further nucleic acid amplification reaction after removing the hydrophobic solvent, a copy number of amplified nucleic acid fragments of about 1000 to 10,000 molecules may be obtained.

### Example 6

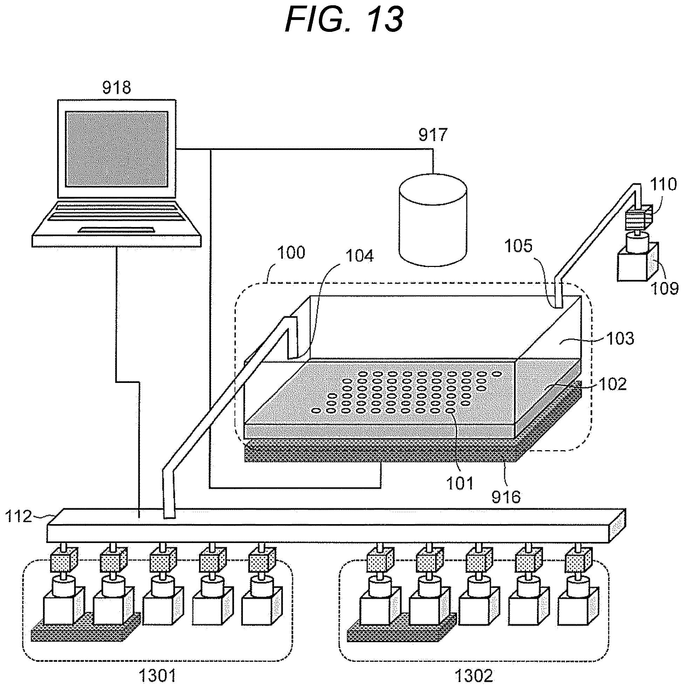

FIG. 13 is a configuration diagram of an example of an apparatus for nucleic acid analysis when DNA sequencing is performed. Specifically, a DNA sequencing reagent set 1302 may be included in addition to the configuration of the nucleic acid analysis apparatus according to the present invention (reagents are collectively represented as an on-substrate nucleic acid cluster forming reagent set 1301). Functions required in the reaction of nucleotide sequence analysis may be solution supply and solution replacement to the flow cell solution tank, temperature control of the flow cell solution tank, and the observation unit. The functions can be shared with the configuration of DNA cluster formation. With the configuration illustrated in FIG. 13, following the DNA cluster formation on the substrate 102, it may be possible to analyze the nucleotide sequence with the same flow cell solution tank 103 as a reaction field without moving the substrate 102. Further, the configuration including the observation unit 917 and the control unit 918 may enable automation of control and observation of the series of steps.

The present invention is not limited to the examples described above, and includes various modifications. For example, the above examples are described in detail to easily understand the present invention, and the invention is not necessarily limited to the examples having all configurations described above. In addition, a portion of the configuration of an example can be substituted by the configuration of another example, and also, the configuration of another example can be added to the configuration of an example. Also, another configuration can be added to, removed from, and substituted for a portion of the configuration of each example.

### Reference Signs List

- 100: flow cell
- 101: hydrophilic pattern region
- 102: substrate
- 103: flow cell solution tank
- 104: first opening
- 105: second opening
- 106: sample solution tank
- 107: hydrophobic solvent tank
- 108: reaction solution tank
- 109: drainage tank
- 110: valve
- 112: pump
- 201: lower layer substrate
- 202: partition wall
- 203: molecules for fixing template nucleic acid
- 305: droplets
- 308: amplified nucleic acid fragments
- 310: sample solution
- 320: hydrophobic solvent
- 330: reaction solution
- 501: hydrophilic pattern region having amplified nucleic acid fragments
- 840: mixed solution of sample solution and reaction solution
- 913: solution mixing tank
- 914: washing solution tank
- 915, 916: temperature control devices
- 917: observation unit
- 918: control unit
- 1301: on-substrate nucleic acid cluster formation reagent set
- 1302: DNA sequencing reagent set

## Claims

1. A nucleic acid amplification method comprising the steps of:
preparing a substrate having a plurality of first surfaces having hydrophilicity and a second surface surrounding each of the plurality of first surfaces, the second surface being less hydrophilic than the first surfaces, wherein a molecule that specifically binds to a template nucleic acid is fixed or arranged on the first surfaces;
supplying a mixed solution of a sample solution comprising the template nucleic acid and a reaction solution comprising a nucleic acid amplification substrate and a nucleic acid synthetic enzyme on the substrate to arrange the mixed solution on the first surfaces, and bind the template nucleic acid to the molecule that specifically binds to the template nucleic acid;
supplying a hydrophobic solvent on the substrate to form droplets in which the mixed solution arranged on the first surfaces is enclosed;
performing an amplification reaction of the nucleic acid in the droplets;
removing the hydrophobic solvent from the substrate;
supplying a reaction solution comprising a nucleic acid amplification substrate and a nucleic acid synthetic enzyme on the substrate; and
performing an amplification reaction of the nucleic acid.

2. The nucleic acid amplification method according to claim 1, wherein the amplification reaction is rolling circle amplification (RCA) or polymerase chain reaction (PCR).

3. The nucleic acid amplification method according to claim 1, wherein the sample solution comprising the template nucleic acid is diluted to form droplets in which one molecule or less of the template nucleic acid is enclosed per droplet.

4. The nucleic acid amplification method according to claim 1, wherein the nucleic acid is selected from the group consisting of messenger RNA (mRNA), non-coding RNA (ncRNA), microRNA, genomic DNA, fragments thereof, and hybrid nucleic acids of RNA and DNA.

5. The nucleic acid amplification method according to claim 1, further comprising, after the step of performing an amplification reaction of the nucleic acid in the droplets, the steps of:
supplying a mixed solution of a sample solution comprising the template nucleic acid and a reaction solution comprising a nucleic acid amplification substrate and a nucleic acid synthetic enzyme on the substrate to arrange the mixed solution on first surfaces that do not comprise an amplified nucleic acid fragment among the first surfaces, and bind the template nucleic acid to the molecule that specifically binds to the template nucleic acid;
supplying a hydrophobic solvent on the substrate to form droplets in which the mixed solution arranged on the first surfaces is enclosed; and
performing an amplification reaction of the nucleic acid in the droplets.

6. The nucleic acid amplification method according to claim 1, further comprising the step of drying the substrate, after the step of removing the hydrophobic solvent from the substrate.

7. An apparatus for nucleic acid analysis comprising:
a reaction vessel in which a nucleic acid amplification reaction is performed;
a sample solution tank for storing a sample solution comprising a template nucleic acid;
a hydrophobic solvent tank for storing a hydrophobic solvent; and
a reaction solution tank for storing a reaction solution comprising at least a nucleic acid amplification substrate,
wherein the reaction vessel is provided with a first opening and a second opening that are connectable to the sample solution tank, the hydrophobic solvent tank, and the reaction solution tank;
a substrate that has a plurality of first surfaces having hydrophilicity and a second surface surrounding each of the plurality of first surfaces is provided on either a top surface or a bottom surface in the reaction vessel, the second surface being less hydrophilic than the first surfaces; and
a molecule that specifically binds to a template nucleic acid is fixed or arranged on the first surfaces.

8. The apparatus for nucleic acid analysis according to claim 7, wherein the shape of the substrate has a concave structure, and the bottommost surface of the concave structure is the first surface.

9. The apparatus for nucleic acid analysis according to claim 7, wherein the first surfaces have a diameter of 0.5 to 2.0 µm, and the density of the first surfaces on the substrate is 200,000/mm² or more.

10. The apparatus for nucleic acid analysis according to claim 7, further comprising:
an observation unit capable of observing the substrate, and/or
a drainage tank for collecting drainage from the reaction vessel.

11. The apparatus for nucleic acid analysis according to claim 7, further comprising at least one temperature control device.

12. The apparatus for nucleic acid analysis according to claim 11, wherein the temperature of the reaction vessel, the sample solution tank, the hydrophobic solvent tank, and the reaction solution tank can be adjusted to an appropriate temperature by the temperature control device.
